Europäisches Patentamt

European Patent Office

Office européen des brevets

(11) Publication number: **0 376 080**
**A1**

(12) **EUROPEAN PATENT APPLICATION**

(21) Application number: **89123171.4**

(22) Date of filing: **14.12.89**

(51) Int. Cl.5: **C12N 15/10, C12P 19/34**

(30) Priority: **22.12.88 IT 8355188**

(43) Date of publication of application:
**04.07.90 Bulletin 90/27**

(84) Designated Contracting States:
**AT BE CH DE ES FR GB GR LI LU NL SE**

(71) Applicant: **Talent SRL**
**Via del Follatoio 12**
**I-34148 Trieste(IT)**

(72) Inventor: **Schneider, Claudio**
**Via Reana 20**
**I-33100 Udine(IT)**

(74) Representative: **Petraz, Gilberto Luigi**
**G.L.P. S.a.s. di Gilberto Petraz P.le Cavedalis**
**6/2**
**I-33100 Udine(IT)**

(54) **Method for the extraction and purification of DNA.**

(57) Method for the extraction and purification of
DNA, starting from lambda phages, M13 phagemids,
plasmids, cosmids or other like elements, which
provides for three steps:
- charging the sample, which undergoes an exchange treatment, or lysis, followed by macrofiltration,
- the macrofiltrate is treated with a precipitant and
the resulting solution is subjected to ultrafiltration,
- the DNA fixed to the filtration support of ultrafiltration is eluted and collected.
    Lambda DNA, single-stranded DNA from
phagemids, plasmidic DNA and cosmidic DNA ob-
tained by the above method.

EP 0 376 080 A1

## METHOD FOR THE EXTRACTION AND PURIFICATION OF DNA

This invention concerns a method to extract and purify DNA. To be more exact, the invention provides a method which can be reproduced automatically with modest execution times and with simple automation problems.

The invention can be employed to obtain DNA from lambda phages or the like, M13 phagemids and the like, and also from plasmids and cosmids and the like.

The journal Nucleic Acids Research, vol.15, number 23, 1987, includes on page 10047 an article headed, "A simple and fast method for preparing single-stranded DNA template suitable for sequencing".

The same journal in vol.16, number 7, 1988 includes on page 2873 another article headed, "A new and fast method for preparing high lambda DNA suitable for sequencing".

These articles teach the obtaining of DNA from phagemids and lambda phages respectively.

The methods disclosed in the above articles provide for at least one centrifuging step, and it is the inclusion of these centrifuging steps besides other factors which makes the automation of the method hard and inconvenient.

The above proposed methods arrange also to exchange the detergent CTAB (cetyl-trimethyl-ammonium bromide) with the sodium ion by means of re-suspension of the CTAB-DNA pellet, obtained after centrifuging, in a 1.2 M NaCl solution with the additon of two volumes of ethanol and further centrifuging.

Although these teachings have improved the state of the art by eliminating the separation of steps inherent in deproteinization by means of phenol, yet they entail the shortcoming of not enabling the process of obtainment to be automated simply, for the centrifuging in fact hinders or, in any event, makes very complex the automation of the process except with excessive costs and unjustified execution times.

To obviate these shortcomings and achieve further advantages which will become clear in the following description, such as the speed of the process and the ability to reproduce the results, as well as the yield, the present applicant has studied, tested and obtained the present method.

The method according to the invention is set forth in the main claim, whereas the dependant claims describe variants of the idea of the main solution.

The invention provides for the use of appropriate, differentiated filtration cycles instead of centrifuging.

The method according to the invention enables plasmid DNA, cosmid DNA, phagemid DNA or lambda phage DNA to be obtained.

The DNA obtained with the method can be employed in further analyses of:

1) enzymatic restriction

2) cloning

3) enzymatic synthesis reactions (RNA polymerase and DNA polymerase)

4) sequencing reactions.

The yield of DNA by percentage, as compared to the methods described in the aforesaid publications making use of centrifuging, ranges from 80% to 100%.

According to the invention differentiated filtrations with differentiated functions are employed to obtain the required DNA.

The method according to the invention comprises at least the following steps:

step 1: charging the sample, which undergoes a first exchange treatment followed by macrofiltration.

According to the invention the exchange treatment can be differentiated in relation to the typology of the starting sample;

step 2: the macrofiltrate is treated with a precipitant and the resulting solution is subjected to ultrafiltration;

step 3: after washing, the DNA fixed to the filtration support is eluted.

Let us examine these individual steps one by one.

## Step 1.

After the charging, an anionic exchange resin (solution A), DEAE resin for instance, in a quantity of a volume equal to that of the sample, is added to the sample of lysate of phages or phagemids. Another analogous substance can be used instead of the anionic exchange resin.

The whole is re-mixed for a time long enough for the desired exchange to take place. This time may be 10 minutes at the ambient temperature, for instance.

For the bacterial culture, in order to obtain plasmidic DNA or cosmidic DNA the solution A which is added will contain an alkaline solution of lysis (ph $\geqslant$ 11) that comprises sodium desoxycolate (DOC) ($\geqslant$ 0.05%) or another like product.

After the necessary re-mixing for proper homogenization of the solution thus produced (mixing lasting for 2 or 3 minutes, for instance) at the ambient temperature a solution (solution B) containing an acid, acetic acid for instance, is added to the bacterial culture alone so as to bring the pH to about 5 (± 2).

The solution thus produced is mixed again for enough time to obtain formation of a macroflocculate (complex of DOC/genomic DNA/cellular debris).

The invention provides for the use of desoxycolate (DOC) as a detergent for bacterial lysis, instead of dodecylsulphate (SDS) as taught in the state of the art when the sample contains plasmid or cosmid, for the exchange treatment.

The essential advantage of the use of detergent DOC in the process is the more efficient precipitation of the detergent/ genomic DNA/cellular debris complex with a slightly acid pH (pka of DOC = 6.9 whereas the pka of SDS = 1-2).

Precipitation of the detergent in an acid ambient ( < 6.9) enables the detergent and that which is associated with it to be wholly removed, as opposed to what happens when SDS is employed.

In fact, with the detergent SDS used in the most common processes to obtain plasmidic DNA or cosmidic DNA it is necessary, besides acidification, to increase the concentration of sodium ions and to lower the temperature.

If SDS is used to proceed thereafter to the step of precipitation with CTAB, as described in the cited state of the art, it is necessary to dilute the filtrate by three times beforehand.

Moreover, even when the filtrate is diluted by three times, the detergent SDS, if employed to produce the solution, is not wholly eliminated, and this causes a successive inefficient formation of the DNA-CTAB complex.

With macrofiltration through a macroporous diaphragm the DEAE resin is separated out with all the anionic impurities associated therewith in the case of a lysate of phages/phagemids and the macroflocculate is separated out in the case of a bacterial culture (plasmids or cosmids).

In fact, depending on the starting element, the impurities will have been adsorbed by the exchange resin DEAE or will be the object of macroprecipitate generated by the DOC detergent of lysis.

The macroporous diaphragm may consist, for instance, of compressed gauze or an equivalent filter means.

In the case of lysate of phages or phagemids the macroporous diaphragm may be supported advantageously by a nitrocellulose filter, for instance, of an 0.2 micron type or the like (Teflon, borosilicates, etc.).

## Step 2.

To the filtrate is added the precipitant solution (solution C) containing, for instance, cetyl-trimethyl-ammonium bromide (CTAB), which is a cationic detergent of a low ionic force that forms a micellar complex with the polyanion DNA.

This precipitant solution enables the DNA/CTAB micellar complex to be formed after brief mixing for homogenization.

Where plasmids or cosmids are used as the starting material, the macrofiltrate can be neutralized beforehand and be treated with an enzyme, RNasi A, for instance, to digest the RNA contained in the lysate before addition of the precipitant.

The solution thus obtained and containing the micellar complex then undergoes ultrafiltration.

The ultrafiltration can be carried out with any known microporous support; for instance, a microporous support consisting of borosilicate glass (DURAN type or the like with a porosity of 4) has been usefully employed and has enabled the DNA-CTAB micellar complex to be retained satisfactorily.

The second filtration, being of an ultrafiltration and inert type, makes possible:

1) adhesion of the DNA-cationic detergent (CTAB for instance) micellar precipitate;

2) washing and exchange of the detergent with the ions, Na for instance,

3) recovery of the DNA in the form of salt, for instance sodium salt, in an aqueous solution.

## Step 3.

The DNA (lambda, phagemid, plasmid, cosmid) being now immobilized on the filter (as a result of Step 2), it is now eluted.

However, this elution takes place after a series of washings of the filter, as follows for instance:

- a first wash is carried out in an aqueous solution of a low ionic force to eliminate excess detergent;

- a second wash is carried out with a mixture of alcohol and ionic solution, for instance of a NaCl 0.2 M/70% ethanol type, to exchange the DNA-CTAB into DNA-Na;

- a third wash is then carried out with a mixture of alcohol and a solution of a low ionic force to remove the excess salts, for instance of a NaCl type (for example, 70% ethanol, 30% water).

After having dried every trace of alcohols carefully from the filter, the DNA is then recovered by washing the filter with an aqueous solution of a low ionic force (water for instance).

The DNA thus obtained, whether lambda DNA, single-stranded DNA from phagemids or plasmidic DNA or cosmidic DNA, can now be used for subsequent purposes.

## Claims

1 - Method for the extraction and purification of DNA, starting from lambda phages, M13 phagemids, plasmids, cosmids or other like elements, characterized in that it provides for three steps:
- charging the sample, which undergoes an exchange treatment, or lysis, followed by macrofiltration,
- the macrofiltrate is treated with a precipitant and the resulting solution is subjected to ultrafiltration,
- the DNA fixed to the filtration support of ultrafiltration is eluted and collected.

2 - Method as claimed in Claim 1, in which in the case of lambda phages and M13 phagemids the exchange treatment is carried out with an anionic exchange resin, DEAE for instance.

3 - Method as claimed in Claim 1, in which in the case of plasmids or cosmids an alkaline solution of lysis (pH⩾ 11) is added which contains a detergent, for instance sodium desoxycolate (DOC).

4 - Method as claimed in Claim 1 or 3, in which a solution containing acid is added to bring the pH to about 5 (± 2) so as to obtain a macroflocculate (for instance, DOC/genomic DNA/cellular debris).

5 - Method as claimed in any claim hereinbefore, in which the macrofiltration takes place with a macroporous diaphragm.

6 - Method as claimed in any of Claims 1, 2 or 5, in which in the case of lambda phages or M13 phagemids the macroporous diaphragm is supported by a nitrocellulose filter of 0.2 microns or the like.

7 - Method as claimed in any of Claims 1, 3, 4 or 5, in which the macrofiltrate derived from plasmids or cosmids is neutralized at least to reduce the acidity.

8 - Method as claimed in any claim hereinbefore, in which the macrofiltrate is treated with an enzyme suitable to digest RNA.

9 - Method as claimed in any claim hereinbefore, in which a precipitant solution containing a cationic detergent, for instance CTAB (cetyl-trimethyl-ammonium bromide), is added to the macrofiltrate to obtain a micellar complex.

10 - Method as claimed in any claim hereinbefore, in which the micellar complex undergoes ultrafiltration, successive washing and elution of the retained portion with recovery of the DNA.

11 - Lambda DNA, single-stranded DNA from phagemids, plasmidic DNA and cosmidic DNA obtained according to one or another of the features of the claims hereinbefore.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | NUCLEIC ACIDS RESEARCH, vol. 16, no. 7, 11th April 1988, pages 2873-2884, IRL Press Ltd, Oxford, GB; G. MANFIOLETTI et al.: "A new and fast method for preparing high quality lambda DNA suitable for sequencing" * Materials and method" * --- | 1 | C 12 N   15/10 C 12 P   19/34 |
| A | EP-A-0 240 191  (SEIKO INSTRUMENT INC.) * Page 1, column 1, line 35 - column 2, line 27 * --- | 1 | |
| A,D | NUCLEIC ACIDS RESEARCH, vol. 15, no. 23, 1987, page 10047, IRL Press Ltd, Oxford, GB; G. DEL SAL et al.: "A simple and fast method for preparing single stranded DNA template suitable for sequencing" ----- | | |

**TECHNICAL FIELDS SEARCHED (Int. Cl.5)**

C 12 N
C 12 P

The present search report has been drawn up for all claims

| Place of search | Date of completion of the search | Examiner |
|---|---|---|
| THE HAGUE | 23-03-1990 | VAN PUTTEN A.J. |